# EUROPEAN PATENT SPECIFICATION

(11) **EP 3 038 664 B1**
(45) Date of publication and mention of the grant of the patent: **11.03.2020**
(21) Application number: 14761743.5
(22) Date of filing: 20.08.2014
(51) Int. Cl.: A61L 27/36, A01N 1/02, A61F 2/24, A61F 2/00

(54) **TRANSCATHETER VALVE WITH LYOPHILIZED TISSUE**
TRANSKATHETERVENTIL MIT LYOPHILISIERTEM GEWEBE
VALVE DE TRANSCATHÉTER À TISSU LYOPHILISÉ

(30) Priority: 29.08.2013 US 201361871356 P
(43) Date of publication of application: 06.07.2016
(73) Proprietor: St. Jude Medical, Cardiology Division, Inc., St. Paul, MN 55117 (US)
(72) Inventor: VENKATASUBRAMANIAN, Ramji T., Maple Grove, Minnesota 55311 (US); AHMANN, Katherine A., Minnesota 55112 (US)
(74) Representative: Gill Jennings & Every LLP
(86) International application number: PCT/US2014/051856
(87) International publication number: WO 2015/031124

(56) References cited:
- WO-A2-02/053069
- WO-A2-2007/076508
- DE-T2- 60 022 075
- US-A1- 2006 155 358
- US-B2- 8 105 375

## Description

### CROSS-REFERENCE TO RELATED APPLICATIONS

The present application claims the benefit of the filing date of U.S. Provisional Patent Application No. 61/871,356 filed August 29, 2013.

### BACKGROUND OF THE INVENTION

The human body contains a number of valves, perhaps best known among those are the valves of the heart. These valves can suffer from congenital conditions or disease that can force their repair or replacement. A number of replacement valves exist, some synthetic and some made with flexible tissue of biologic origin. The latter valves, known herein generally as "tissue valves", pose several challenges. First, tissue used must not cause an immune response when implanted into a human or animal patient. Second, the tissue and indeed the entire valve should be sterile. Third, tissue valves must be storage stable from manufacture until use. And, the tissue used must be suitably durable to withstand prolonged use and millions of cycles of opening and closing under circulatory pressure without failure.

The complex requirements placed on the tissue used in valves is amplified as new generations of "collapsible" tissue valves are developed. Some new valves may be implanted using more minimally invasive surgical techniques such as a trans-apical or trans-femoral technique. Such valves, including the recently described transaortic valve replacement ("TAVR") valves, place the same performance requirements on the tissues as other tissue valves. But, in addition, the tissues used must be capable of being compressed into a small enough profile to allow a transvascular introduction. And, they must be robust enough to withstand the stresses that may come from being mounted onto a self expanding or balloon expandable stent and implanted using relevant techniques.

Techniques that can assist in production of tissue valves with improvements in any of these properties, improved production cost, and/or improved ease of use, are desirable and sought after.

Chemical dehydration and lyophilization of heart tissue used in valves tissue are known in the art. Some disclosures relating to either dehydration or lyophilization can be found in U.S. Patent Nos. 8,105,375; 8,007,992; 6,277,555; 6,534,004 and U.S. Patent Appln. Nos. 13/189,036 and 13/038,361.

### BRIEF SUMMARY OF THE INVENTION

The invention as defined in claim 1 is broadly directed to methods of making tissue valves or components thereof, wherein at least some of the tissue is prepared by lyophilization (freeze drying). Lyophilization of the tissue can occur before or after construction of a valve assembly or before or after assembly of the tissue valve. Thus in one embodiment, "wet" tissue (bioprosthetic tissue prior to lyophilization) is attached to a support to construct a valve assembly on the support and thereafter the entire resulting tissue valve is lyophilized. In another embodiment, the "wet" tissue is used to construct a "wet" valve assembly. This "wet" valve assembly is attached first and the entire resulting tissue valve lyophilized.

For clarity, the term "valve" as used herein refers to a complete and operable structure capable of being implanted into a patient in need thereof. The terms "implanted" or "implantation" as used herein refers to a complete and long-term seating of a valve in a patient. A valve can be a surgical valve, a TAVR valve or any other valve structure. A "tissue valve" is a valve that includes at least some tissue of biologic origin (also known herein and generally as "bioprosthetic tissue". A "valve assembly" as used herein is a structure that is made from, at least in part, tissue, and operates to meter or restrict fluid flow for at least some period of time, but does not include other structures like a stent often used to support the valve assembly. Thus, a tissue valve for present purposes is a valve that includes a valve assembly comprised of at least some bioprosthetic tissue. The tissue valve may, and often does, include other structures, such as a supporting stent. Valves in accordance with the present invention may be used in the heart as replacements for one of the native valves found therein, such as the aortic or mitral valves. But these valves are not limited thereto and can be used in other vessels, including blood vessels.

An example of a method of making a tissue valve includes attaching a valve assembly comprised of at least one leaflet to an interior surface of a support configured to fit within at least one blood vessel. The method also includes lyophilizing the valve assembly. According to the invention, attaching is performed before lyophilizing. In some embodiments, the resulting lyophilized valve assembly may have a moisture content of about 10% or less, 5% or less, or 1% or less. Further, the lyophilized valve assembly may include about 5% or less, 2% or less, or 1% or less residual organic solvent by weight of the lyophilized valve assembly. In some embodiments, the valve assembly may also include at least one cuff, where the at least one leaflet and cuff are separately attached to the interior surface of the support. In one embodiment, the valve assembly may be lyophilized after the at least one leaflet and cuff are attached to an interior surface of the support. Any lyophilization technique can be used. In one embodiment, the tissue is lyophilized in a procedure that involves washing/soaking bioprosthetic tissue in a solution, such as in saline, with or without a lyoprotectant or cryoprotectant, followed by lyophilization. Any bioprosthetic tissue may be used including, without limitation, tissue that has been cross linked or "fixed" such as by use of glutaraldehyde and/or treated to reduce calcification.

In one aspect, the tissue valves are those produced by any of the processes described herein. In particular, a tissue valve comprising a support and an attached valve assembly, either created on the support from "wet" tissue components or a fully assembled "wet" valve assembly, wherein the entire resulting tissue valve was lyophilized is contemplated. So too is a tissue valve comprising a support and an attached valve assembly created on the support from either previously lyophilized tissue or a previously lyophilized valve assembly.

An example of a tissue valve includes a stent configured to fit within at least one blood vessel and have a collapsed configuration, an expanded configuration and an interior surface. A lyophilized valve assembly is attached to the interior surface and has at least one leaflet having a moisture content of about 10% or less, 5% or less, or 1% or less. In one embodiment, the tissue valve may be configured to fit within a delivery device having a smaller diameter when in the collapsed configuration relative to the diameter of an identical valve without a lyophilized valve assembly. In another embodiment, the tissue valve may be configured to be partially unsheathable and resheathable from the delivery device. In yet another embodiment, the valve assembly may include at least one cuff, and the at least one leaflet and cuff may be separately attached to the interior surface of the stent. The valve assembly cuff may also have a moisture content of about 10% or less, 5% or less, or 1% or less by weight.

Any of the forgoing tissue valves sterilized and packaged for shipping and/or implantation are also contemplated. Indeed, because the tissue valves do not need to be shipped or stored in solution to prevent the tissue from drying out, the tissue valves may be pre-loaded onto delivery devices with the entire assembly being provided in sterile packaging such that they are able to be reconstituted either before use or *in situ* by blood or other fluids in the patient's body (i.e., biofluids) during implantation. In some embodiments, these lyophilized tissue valves are, by virtue of being lyophilized, loaded onto a smaller diameter delivery device than could be used to deliver an otherwise identical "wet" tissue valve. Alternatively, such valves, if not preloaded, are capable of being loaded onto such a relatively smaller diameter delivery device.

An example of a packaged tissue valve ready for reconstitution includes a package having an outer periphery that defines an inner-space. The inner-space has an environment that is substantially dry and sterile. The tissue valve includes a support configured to fit within at least one blood vessel and a lyophilized valve assembly attached to an inner surface of said support. The dry tissue valve is encased within the package. In one embodiment, the packaged tissue valve may include a delivery device. In another embodiment, the packaged tissue may be preloaded within the delivery device.

In another aspect, partial or complete reconstitution of any of the foregoing tissue valves is contemplated. In one embodiment, the tissue valve comprises a lyophilized valve assembly attached to a stent that is capable of being resheathed, with the resulting tissue valve loaded onto a delivery device which will permit resheathing. The support and the valve assembly are configured cooperatively such that the valve assembly can be at least partially exposed when on a delivery device, at least partially reconstituted, sheathed/resheathed and/or implanted. This allows the surgical team to either expose the valve assembly for at least partial reconstitution prior to implantation or within the patient during implantation. In a further aspect of this embodiment, the support is structured such that the valve assembly can be exposed for at least partial reconstitution without completely exposing the balance of the support. Complete reconstitution while partially exposed is also contemplated. In a further embodiment, a tissue valve which is not preloaded onto a delivery device is at least partially reconstituted and thereafter crimped and loaded onto a delivery device.

Alternatively, methods of reconstituting a tissue valve comprised of lyophilized tissue *in situ* (in the patient) are contemplated without falling under the invention. Such methods comprise the steps of introducing the valve loaded onto a delivery device into a vessel of a patient in need thereof, partially unsheathing the tissue valve in the patient such that substantially all of the valve assembly is exposed for a time sufficient to allow the valve assembly to at least partially reconstitute, and implanting the tissue valve. In one further embodiment, the operability of the valve assembly is checked or assessed during or after at least partial reconstitution but before implantation. In another embodiment, the tissue valve is fully reconstituted after implantation.

In yet another embodiment, the valve assembly may be at least partially reconstituted while fully sheathed. Partial reconstitution while fully sheathed may be achieved by irrigating solute through the delivery device prior to implantation.

### BRIEF DESCRIPTION OF THE DRAWINGS

These and other features, aspects, and advantages of the present invention will become better understood with regard to the following description, appended claims, and accompanying drawings where:
FIG. 1 is an exemplary TAVR tissue valve that may include a lyophilized valve assembly.
FIG. 2 is flow chart of various embodiments not falling under the invention of a method of making the TAVR tissue valve of FIG. 1.
FIG. 3 is flow chart of various embodiments of a method falling under the invention of making the TAVR tissue valve of FIG. 1.
FIG. 4 is a flow chart of alternative methods of packaging the TAVR tissue valve for shipping and implantation in either a pre-loaded configuration or a free-standing configuration.
FIG. 5 is a flow chart of various embodiments of a method of reconstituting the TAVR tissue valve in the pre-loaded configuration which does not fall under the invention.
FIG. 6 is a flow chart of various embodiments of a not in accordance with the invention of reconstituting the TAVR tissue valve in the free-standing configuration.

### DETAILED DESCRIPTION

Lyophilization of various forms of tissue for use in prosthetic heart valve structures, examples of which are described herein, offers a number of possible advantages. Because of the reduction in liquid, the resulting tissue valve may be capable of assuming a relatively smaller size compared to the same valve with "wet" tissue, potentially allowing it to be delivered by a smaller diameter catheter. The fact that the bioprosthetic tissue is lyophilized may also facilitate "crimping" onto a delivery device by the manufacturer. This can also result in the ability to use a smaller delivery catheter because exact and consistent crimping and loading techniques can be employed to provide an optimal configuration. This consistent process could allow for a reduction in operator error and can significantly increase convenience for the surgical team.

Further, a prosthetic valve with lyophilized tissue may not need to be stored and/or shipped in a sterilizing and hydrating solution which may make manufacture and transport less expensive, storage and use more convenient, and may result in less chemical waste to dispose. Additionally, the lyophilization process may allow for chemical preservatives and dehydrants to be eliminated from use altogether or, if used, to be significantly eliminated from the tissue either prior to lyophilization or during the lyophilization process. Thus, lyophilization is capable of providing additional assurances that the lyophilized tissue may be introduced to the patient substantially free of, for example, dehydrating chemicals.

Indeed, because such tissue valves do not need to be shipped or stored in solution to prevent the tissue from drying out, the tissue valves may be pre-loaded onto delivery devices with the entire assembly being provided in sterile packaging such that they are able to be immediately reconstituted either before use or *in situ* by blood or other fluids in the patient's body during implantation.

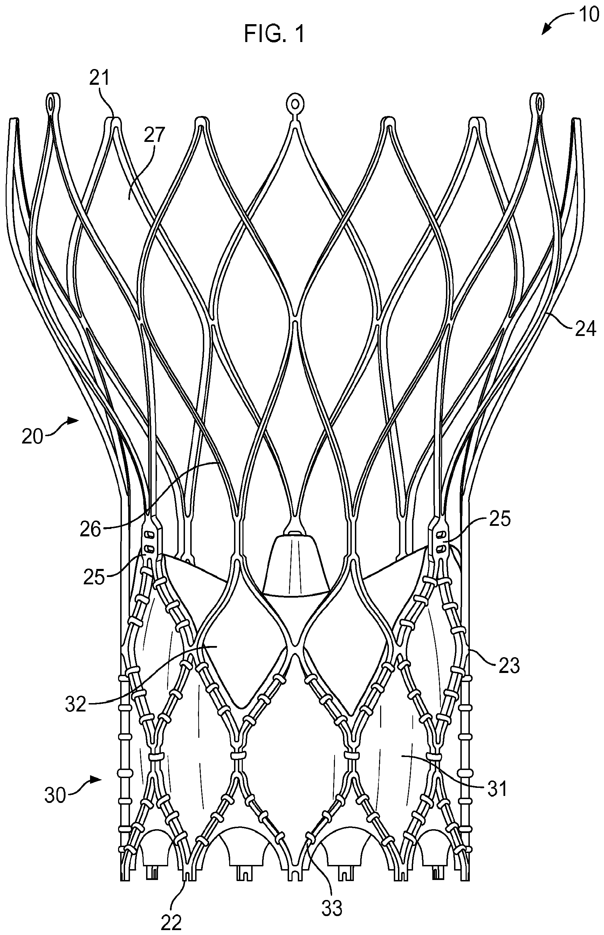

FIG. 1 depicts an exemplary TAVR tissue valve 10 that includes a stent 20 and valve assembly 30. The valve assembly 30 may include a cuff 31 and at least one leaflet 32. In this particular illustration, the leaflet 32 is affixed to the cuff 31 by being sewn to the cuff 31 via a suture (not shown). Multiple leaflets 32 may also be affixed to the cuff 31 in this fashion to form a valve assembly 30. In the illustrated instance, three semi-lunar leaflets 32 are provided as part of valve assembly and this structure imitates the structure of the native aortic valve and is suitable for replacing a patient's native aortic valve. In other arrangements, the leaflets 32 may be attached to the structure of stent 20 through sutures sewn through the cuff 31. It is also possible that both cuff 31 and leaflets 32 are attached to the stent 20 such as at the commissure features 25.

The valve assembly 30 may be made from any material suitable for use in the construction of valves. But for present purposes, it will include at least some tissue. Thus, the valve assembly 30 could be made completely of tissue or combinations with various synthetics. Bioprosthetic tissue must not cause an adverse immune response, must be suitable for its intended purpose and construction, and must be susceptible of lyophilization. Such tissues may originate from any species including, without limitation, cow, pig, horse, dog, primate, or, in appropriate circumstances, may be human. Specific tissues that may be used include, again without limitation, any blood vessel, pericardial tissue, heart muscle tissue, dura mater, and the like. More than one species and tissue type may be used in a valve assembly.

Tissue, either as a part of a tissue valve, valve assembly, or the individual components of a valve assembly may be lyophilized such that the resulting lyophilized tissue has a residual moisture content of about 10% by weight or less of the "dry" tissue. Moisture content (by weight) can be measured by dividing the difference between the processed tissue weight and the fully dried tissue weight by the processed tissue weight (which can be multiplied by 100 to arrive at a percentage). Processed tissue weight is the weight of the tissue processed by lyophilization as previously described herein (or the "dry" tissue). Fully dried tissue weight is the weight of the tissue that is completely dried of all residual moisture, which can be determined by subjecting a test sample of the "dry" tissue to drying heat within a test chamber of a predetermined volume. In such a procedure, a humidity sensor can measure the moisture lost during the heat drying of the sample tissue. An alternative measurement to moisture content may be percent solids of the tissue. When using such measurement standard, the percent solids of the resulting lyophilized tissue will be about 90% by weight or more, Note that "moisture" in this context refers to water and/or whatever liquid content on and within the tissue is composed of. In some embodiments, the residual moisture content is 5% or less. In another embodiment, the residual moisture content is 1% by weight or less. In some embodiments, the amount of residual organic solvents should be less than 5% by weight of the dry tissue or less. In another, embodiment, the amount of organic solvents present is less than 2% by weight or in other embodiments, less than 1% by weight of the dry tissue,

Any lyophilization technique may be used to convert "wet" tissue, "wet" valve assemblies, and "wet" tissue valves into "dry" tissue, "dry" valve assemblies and "dry" tissue valves. These techniques generally involve freezing the valve assembly followed by drying the frozen valve assembly to promote sublimation.

In some instances, prosthetic tissue used in a valve assembly discussed herein may be treated to crosslink or "fix" the tissue. This can be accomplished by a number of techniques such as those described in US Patent Nos. 7,559,953; 6,547,827; 5,746,775 and 7,918,899. In addition, prosthetic tissue used to produce a valve assembly may be treated to reduce or mitigate calcification. Processes known to mitigate calcification in a bioprosthetic implant tissue include those described in US Patent Nos. 7,972,376; 7,579,381; RE40570; 5,746,775; 7,214,344; 7,029,434; 6,878,168; 6,561,970; and US Patent Publication Nos. 2008/0302372; and 2009/0164005. These treatments can each be performed before or after lyophilization and in any order. However, they are normally performed before lyophilization with fixing occurring before anti-calcification treatment.

The individual components of a valve assembly, such as a leaflet and cuff, may be formed by cryo-cutting, laser cutting, or press cutting the aforementioned materials into the desired shape and thickness. As an example, a few techniques known for cutting and forming tissue can be found in U.S. 7,141,064 and 2011/1328167. This may be performed either before or after lyophilization, but in accordance with the invention before lyophilization.

The stent 20 may include a proximal end 21, a distal end 22, an annulus section 23, an aortic section 24, and a plurality of commissure features 25. Note that the terms "proximal" and "distal" are relative terms and are used herein to mean close to the medical professional (proximal) or further away (distal). Thus, in a trans-apical delivery, the annulus section 23 of the TAVR valve in FIG. 1 may be adjacent to a proximal end (shown as distal end 22), and the aortic section 24 may be adjacent to a distal end (shown as proximal end 21). But, as shown in FIG. 1, the tissue valve 10 is designed for trans-vascular delivery and the annulus section 23 is adjacent the distal end 22 and the aortic section 24 is adjacent the proximal end 21.

The stent 20 may be formed by a plurality of interconnecting struts 26 that define individual cells 27. Stent 20 is collapsible to reduce the overall diameter of the tissue valve 10. As such, the tissue valve 10 may have a collapsed configuration and an expanded configuration and be made from a reversibly deformable material to facilitate collapsibility and expandability. Examples of reversibly deformable material may be a memory metal, such as nitinol, or a shape-memory polymer. In other embodiments, the stent 20 may merely be expandable-not reversibly so. The stent 20 can be made from any conventional technique such as by laser cutting a nitinol tube.

The stent or support structure need not be made from the materials or bear the construction illustrated in FIG. 1. Any size, shape or configuration can be used. These include, without limitation, those illustrated in the following publications all of which describe valves that could be redesigned as tissue valves using lyophilized tissue: Patent Nos. 5,411,552; 6,004,330; 6,168,614; 6,582,462; 6,830,584; 7,018,406; 7,468,073; 7,503,929; 7,914,569; 7,914,575; 7,993,394; 8,002,825; 8,114,155; 8,123,800; 8,206,438; 8,236,045; 8,454,686; 8,454,685; 8,348,996; 8,398,058; U.S. Reissue Patent No. RE42,395; U.S. Publication Nos. 2009/0157175; 2010/0049313; 2012/0089223; 2012/0123529 2012/0296418; 2012/0303113; and AU Publication No. 2010/311811.

The particular structure of stent 20, in combination with valve assembly 30, as illustrated in FIG.1 allow the valve assembly 30 to be exposed and its operation checked before the tissue valve 10 is implanted. If the valve assembly 30 is not fully functioning, the relative position of the tissue valve 10 is suboptimal, or the tissue valve 10 needs to be resheathed within a delivery device for some other reason, this structure permits same. For an example of a resheathable tissue valve *see* U.S. Publication No. 2012/20053681.

Some representative lyophilization conditions that may be used in a various methods for making lyophilized tissue for use in a prosthetic heart valve, such as that shown in Fig. 1 can include: 1) freezing the valve assembly at -20 degrees C for at least 12 hours followed by a single vacuum drying step at, for example, 150 mTorr at room temperature (20-25 degrees C) for 15-20 hours; 2) freezing at -70 degrees C for at least 2 hours followed by annealing (freezing at an increased temperature such as -20 degrees C for at least one hour and then refreezing at -70 degrees C for at least 2 more hours followed by drying at reduced pressure of about 750 mTorr; 3) Freezing to about -40 degrees C followed by annealing as discussed previously and refreezing at about -40 degrees C for at least 2 hours and then two stage drying at -5 degrees C at 160 mTorr followed by secondary drying at 20-25 degrees C and 160 mTorr for at least 2 hours. Various combinations of these and other parameters may also be used, or the processes used in series.

Prior to lyophilization, the tissue may be soaked in a solution. Solutions commonly used for lyophilization of tissue may generally be used. One useful solvent system is saline. Saline solution is generally composed of distilled and/or deionized water and sodium chloride in a concentration ranging from 0.1 to 15 g/l. More specifically, the concentration can range from 7.5 to 10.5 g/l. Isotonic saline is often used. In addition to sodium chloride, the following salts may be used: potassium chloride, calcium chloride, magnesium sulfate, disodium phosphate, sodium bicarbonate, magnesium chloride, sodium phosphate, potassium phosphate, or any combination thereof, for example. Additionally, the saline solution may be a balanced salt solution such as Hank's, Earle's, Gey's or Puck's balanced salt solution or be a phosphate buffered solution.

Treating the tissue with a saline solution or like solution prior to lyophilization may provide certain advantages. One of these advantages is that the solution may assist in leeching organic solvents or residue from the tissue. Thus, a saline solution soak in conjunction with lyophilization can ensure that the bioprosthetic tissue is substantially free of unwanted organic solvents (about a 2% concentration or less). Another advantage of a saline, or like solution, soak is that the tissue may become uniformly saturated with a single, primary liquid. Thus, the lyophilization process can be more precisely tailored to sublimate while providing conditions that mitigate harm to the tissue's microstructure.

The resulting lyophilized tissue will have a residual moisture content consistent with the examples described above. Specifically, the resulting moisture content of the tissue can be about 10% by weight or less of the "dry" tissue, as previously described herein. Alternatively, the resultant lyophilized tissue will be about 90% solids by weight or more. In some examples, the residual moisture content is 5% or less, or the resultant tissue is 95% solids or more. In another example, the residual moisture content is 1% by weight or less, or the resultant tissue is 99% solids by weight or more. In some further examples, the amount of residual organic solvents should be less than 5% by weight of the dry tissue or less. In another, embodiment, the amount of organic solvents present is less than 2% by weight or in other embodiments, less than 1% by weight of the dry tissue, as previously discussed.

As a supplement to the lyophilization process, the bioprosthetic tissue may be treated with a lyoprotectant or a cryoprotectant. Generally any lyoprotectant or cryoprotectant that may mitigate or eliminate the formation of ice crystals during the lyophilization process may be used. This may include, but is not limited to, a solution containing a concentration of sucrose of about at least 2% or a mixture of sucrose and hydroxyl ethylene starch ("HES"), wherein the concentration of sucrose is about at least 1.5% and the concentration of HES is about at least 1.5%, a solution containing a glycol alcohol such as propylene glycol, or a solution of dimethyl sulphoxide ("DMSO").

Generally, the tissue, either as a valve assembly, tissue valve, or individual components of the valve assembly, may be soaked in the lyoprotectant or cryoprotectant until sufficient saturation is reached. Sufficient saturation may be determined by a particular lyoprotectant's or cryoprotectant's diffusion coefficient in relation to the bioprosthetic tissue.

Due to sublimation of moisture from the bioprosthetic tissue during the lyophilization process, the volume of the bioprosthetic material utilized within the tissue valve may decrease. This reduction in volume may surprisingly reduce the loading force associated with loading the "dry" tissue valve into the delivery device.

Loading force typically dictates the minimum size a delivery device can achieve, otherwise loading forces would increase as the size of the delivery device decreases such that it may damage the tissue valve and may make unsheathing/resheathing impracticable. Thus, an advantage of a "dry" tissue valve over a "wet" tissue valve is that the "dry" tissue valve may be loaded into a delivery device having a substantially smaller diameter than if the same tissue valve were "wet". As an example, the profile may be reduced at least 1 Fr from, for example, about 18 or 19 Fr to about 17 or 18 Fr. Further, loading forces may be reduced by at least about 50% and in some instances, at least about 25% and in other instances at least about 10% relative to an otherwise identical tissue valve with a "wet" valve assembly.

Another advantage of reduced loading force is that crimping the valve and loading the "dry" tissue valve into the delivery device may be made less laborious and time consuming. Thus, an employee at the factory may be able to load valves faster and with less complication and medical professionals at the surgical site may also be able to load the valves with less complication.

Reconstitution of the lyophilized tissue before use may be complete reconstitution in which the moisture content of the bioprosthetic tissue is roughly equivalent to the moisture content of the bioprosthetic tissue as it would be in equilibrium with the patient's biofluid *in situ.* In such circumstance, and in some embodiments, the delivery device may be configured to accommodate a resulting increase in volume of the valve assembly, if needed. Alternatively, reconstitution may only be partial in which case the moisture content of the valve assembly may increase relative to its lyophilized state, but less than fully reconstituted. In such circumstance, the delivery device may be configured for a lower profile delivery in which the overall profile of the delivery device may be reduced, in some cases by at least one French ("Fr"), over that of a delivery device carrying a completely reconstituted valve assembly.

In one embodiment, the tissue valve comprises a lyophilized valve assembly attached to a stent that is capable of being resheathed which is loaded onto a delivery device which will also permit resheathing. Examples of such combinations of resheathable valves and delivery devices that permit resheathing can be found disclosed in U.S. Publication Nos. 2012/0078350; 2012/0078352; and 2012/0123528. In a further embodiment, the support and the valve assembly are configured cooperatively such that the valve assembly can be exposed on the delivery device, at least partially reconstituted, and/or checked for operability before being resheathed and/or implanted. This allows the surgical team to either expose the valve assembly for reconstitution prior to implantation or within the patient during implantation and resheath the tissue valve. In a further aspect of this embodiment, the support is structured such that the valve assembly can be substantially completely exposed for reconstitution without completely exposing the balance of the support.

Lyophilization of the bioprosthetic tissue according to the invention occurs after construction of a valve assembly utilizing such tissue or can be undertaken before or after the bioprosthetic tissue or the valve assembly made therewith is attached to the stent or support. Thus in one embodiment, "wet" tissue is attached to a support to construct a valve assembly on the support and thereafter the entire resulting tissue valve is lyophilized to form "dry" tissue (bioprosthetic tissue after lyophilization and having a moisture content of about 10% or less). In another embodiment, the "wet" tissue is used to construct a "wet" valve assembly. This "wet" valve assembly is attached first and the entire resulting tissue valve lyophilized. Finally, individual components of a valve assembly may be lyophilized and either fashioned into a valve assembly which is attached to the support or attached individually to the support to create a valve assembly on the stent. It is also contemplated that only certain components of the valve assembly may be lyophilized while the remaining components are not resulting in a hybrid tissue valve that comprises "dry" tissue components and "wet" tissue components. For example, a cuff may be lyophilized, while the individual leaflets remain "wet".

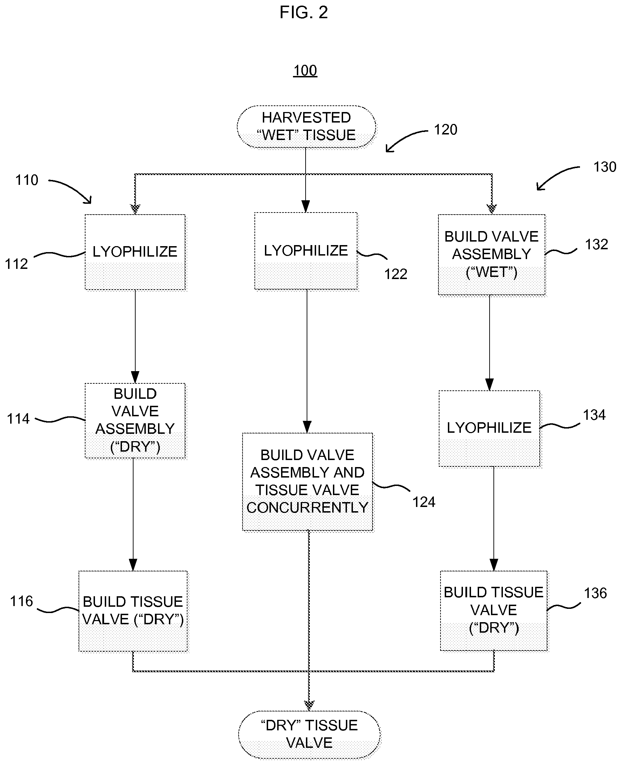

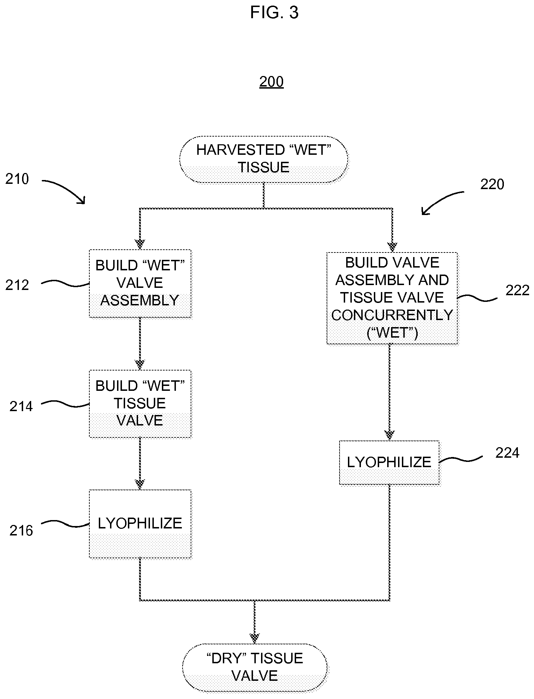

These methods of making a tissue valve that contain "dry" tissue (i.e., a "dry" tissue valve), for example, the TAVR tissue valve of FIG.1, are depicted by the flow charts of FIGS. 2 and 3. FIG. 2 illustrates various embodiments of a method 100 that generally includes lyophilizing "wet" tissue prior to the construction of the "dry" tissue valve. Such "wet" tissue may be lyophilized in the form of a valve assembly or the individual components of the valve assembly. The resulting "dry" tissue may then be used to build a "dry" tissue valve.

The "wet" tissue may be fixed, and optionally washed (not shown). The tissue may also optionally be treated to retard calcification. (not shown) The "wet" tissue can then be cut into the desired shapes for the leaflets and cuff, such as by the techniques discussed above in relation to the exemplary TAVR tissue valve shown in FIG. 1. Cutting could also be undertaken before fixing, after soaking and even following lyophilization in any of these embodiments.

One embodiment 110 of the method as illustrated by FIG. 2 which does not fall under the scope of the claims generally includes lyophilizing the "wet" tissue prior to the formation of a "dry" valve assembly. As such, the individual components (e.g., cuff and leaflets) of the valve assembly may be lyophilized 112 such that their moisture content is about 10% or less. The resulting "dry" tissue leaflets could be sewn to the lyophilized cuff to form the "dry" valve assembly 114 which is then sewn to the stent to form a "dry" tissue valve 116.

Another embodiment 120 not as claimed is depicted in FIG. 2. Embodiment 120 is similar to embodiment 110 in that the "wet" tissue may lyophilized to form "dry" tissue prior to the formation of a valve assembly. However, embodiment 120 differs from embodiment 110 in that the "dry" valve assembly may be formed concurrently with the construction of the "dry" tissue valve by attaching individual structures to the stent or support. Thus, once the formation of the "dry" valve assembly is completed, so too is the "dry" tissue valve. For example, a cuff may be lyophilized 122 and sewn to a stent. Thereafter, individual leaflets may be lyophilized 122 and sewn to the cuff, thereby forming the "dry" valve assembly 124 and "dry" tissue valve. Alternatively, the cuff may be sewn to the stent by at least one suture and thereafter the individual leaflets may be sewn to the stent.

Another embodiment 130 not falling under the scope of claim 1 may include building a "wet" valve assembly from the "wet" tissue prior to lyophilization. Thus, the individual components, while in a "wet" condition, can be sewn 132 together to form a "wet" valve assembly 132. Thereafter, the resulting "wet" valve assembly may be lyophilized 134 to achieve a moisture content of about 10% or less, thereby transitioning the "wet" valve assembly into a "dry" valve assembly 134, which can be stored for future use, or immediately sewn to a support structure (e.g., a stent) to form a "dry" tissue valve 136. This embodiment may be advantageous in that the components of the valve assembly may dry more uniformly than where the individual components are dried in a configuration free from the assemblage. Additionally, the "dry" valve assembly disassociated from the tissue valve support structure may save space when stored for later use.

A method 200 falling under the invention of making a "dry" tissue valve is depicted by the flow chart of FIG. 3. This method 200 generally includes entirely building a "wet" tissue valve prior to lyophilization. Thus, this method excludes affixing bioprosthetic tissue to a support structure or stent 20 while in a "dry" condition. The "wet" tissue utilized in this method may be substantially similar to that utilized in method 100. Substantially similar generally refers the same thickness, stiffness and type of tissue. Geometries of the individual components of a valve assembly may differ between a tissue valve built prior to lyophilization and a tissue valve built after lyophilization to optimize performance and coaptation once complete reconstitution is achieved, particular in examples, described below, that are shipped in a "dry" condition.

In one embodiment 210 falling under the scope of the invention, the "wet" tissue may be in the form of individual components of a valve assembly (e.g., leaflets and cuff). The "wet" tissue may be built into a "wet" valve assembly 212 by sewing each individual leaflet to a cuff. The resulting "wet" valve assembly is then attached to a support structure or stent by sewing the cuff and/or leaflets to the stent with at least one suture to form a "wet" tissue valve. This resulting in "wet" tissue valve 214. In method 200, the "wet" tissue valve, once completely built, is lyophilized to form a "dry" tissue valve 216 that includes lyophilized bioprosthetic tissue

In an alternative embodiment 220, also in accordance with claim 1, the "wet" tissue may be affixed to the support structure or stent to form the "wet" valve assembly and "wet" tissue valve concurrently. Thus, the cuff may be sewn to the stent and thereafter the individual leaflets sewn to the cuff while both the cuff and the leaflets are in a "wet" condition. Once the "wet" valve assembly 222 is formed, the "wet" tissue valve is formed simultaneously. As an alternative, the individual leaflets may be sewn directly to the stent via at least one suture or attached at commissure points in order to form the valve assembly. The resulting "wet" tissue valve is then lyophilized to form a "dry" tissue valve 224 that includes lyophilized bioprosthetic tissue.

"Dry" tissue valves made by method 100 may be very similar to "dry" tissue valves made by method 200 in that such valves comprise a support structure and "dry" tissue that forms a valve assembly. However, a "dry" tissue valve that was made by method 200 may have some advantages over valves made by method 100. One such advantage is that it may be easier to cut and form "wet" tissue than "dry" tissue, particularly because dry tissue may not be as pliable and easy to maneuver. As such, it may also be more laborious to build a tissue valve with "dry" tissue than with "wet" tissue. Also, "dry" tissue valves made by method 200 may, on average, achieve a higher level of performance due to the relatively little manipulation of the bioprosthetic tissue in the "dry" condition.

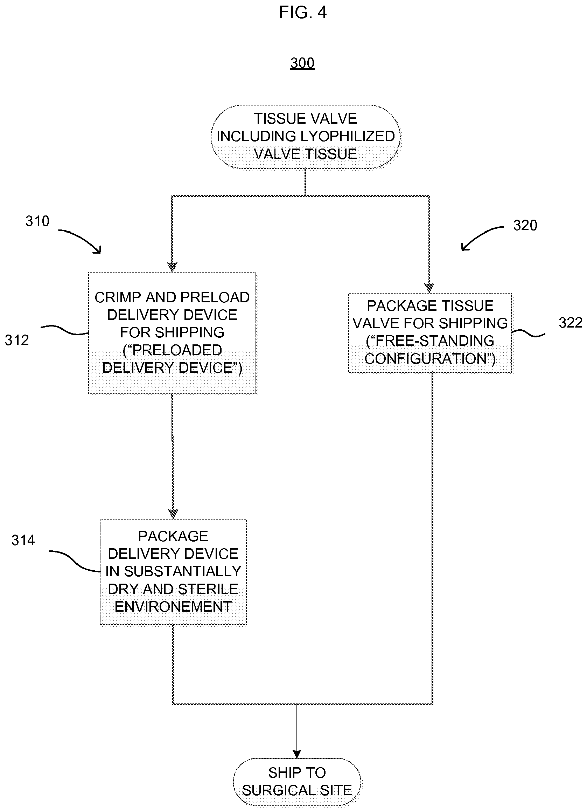

As previously mentioned herein, a "dry" tissue valve may be advantageous in that it may be packaged and shipped free of preserving solution and may be packaged during the manufacturing process in a condition substantially suitable for implantation. Various embodiments of a method 300 of packaging a "dry" tissue valve for delivery and implantation are illustrated by the flow chart of FIG. 4.

In one embodiment 310 of a method 300, a "dry" tissue valve may be crimped and pre-loaded 312 into a delivery device during the manufacturing process. Crimping may be performed in any fashion. In some examples, the forces required for crimping of a "dry" tissue valve may be less than those required for crimping of a "wet" tissue valve.

The preloaded delivery device may be packaged in a container that provides a substantially dry and sterile environment 314 for transit to the surgical site to prevent the reconstitution of the valve tissue and to prevent microbial contamination. This may include the customary package for the delivery device. Additionally, the packaging may be hermetically sealed and filled with an inert gas, such as nitrogen. Such packaging may be in the form of a blister package, for example. In another embodiment 320, the "dry" tissue valve may be packaged 322 in a free-standing configuration. In a free-standing configuration the tissue valve generally has not been crimped and loaded into a delivery device. Thus, the tissue valve may be packaged in a substantially dry and sterile environment (for example, suspended within a glass jar that may further contain a desiccant) almost immediately after the valve is constructed in accordance with one of the previously described methods. So while in this embodiment the tissue valve may not be preloaded into a configuration substantially prepared for implantation, the tissue valve in a free-standing configuration may arrive at the surgical site in a state where the tissue valve may be immediately loaded into a delivery device for implantation.

Sterilization of the bioprosthetic tissue to make suitable for packaging may be performed before and/or after lyophilization. In one example, sterilization can be achieved by chemical solution followed by sterile handling during the lyophilization process. In another example, the bioprosthetic tissue may be exposed to ethylene oxide after lyophilization. In yet another example, sterilization may be performed prior to lyophilization by a chemical process and then sterilized after lyophilization via exposure to a gaseous sterilization agent.

A delivery device suitable for containing a "dry" tissue valve may be a delivery catheter suitable for a trans-femoral or a trans-apical technique, for example. In some instances, the delivery device may allow for the tissue valve to be partially unsheathed and thereafter fully resheathed. Examples of delivery devices that may permit the pre-loading of a "dry" tissue valve and are configured for the tissue valve to be partially unsheathed and implanted via a trans-apical or trans-femoral technique can be found disclosed in U.S. Publication Nos. 2012/0078350; 2012/0078352; and 2012/0123528.

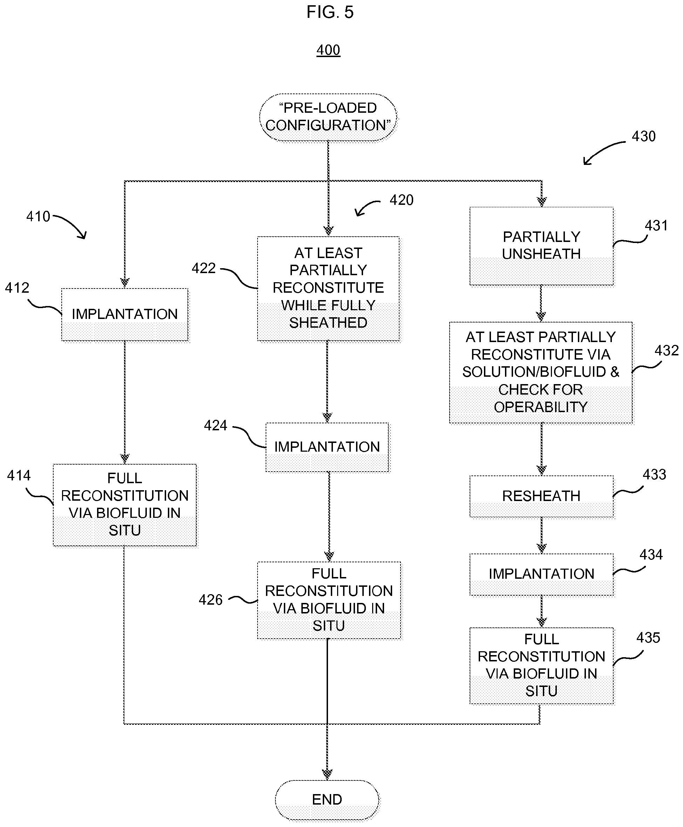

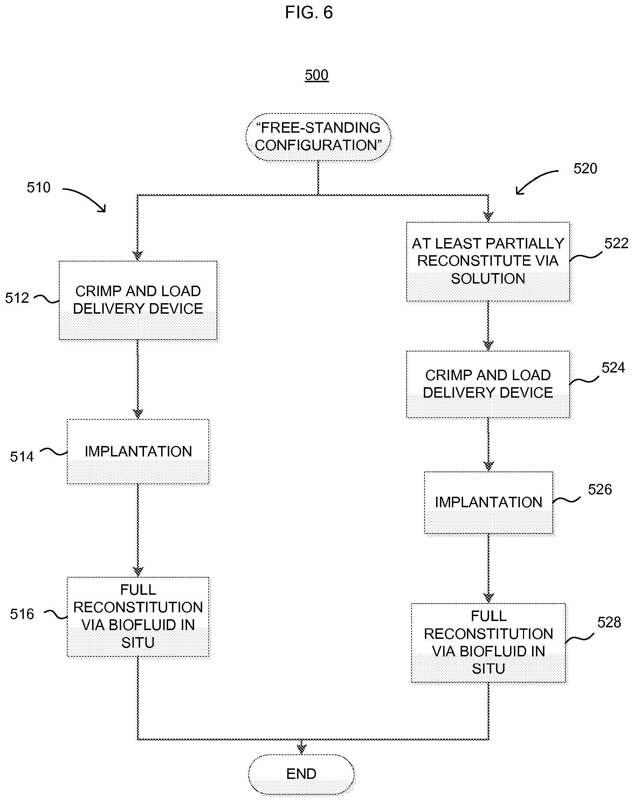

Once the tissue valve arrives at the surgical site in either the pre-loaded configuration or the free-standing configuration, the "dry" tissue valve may be reconstituted. Various reconstitution which are not part of the invention are illustrated by the flow charts in FIGS. 5 and 6. Such methods generally reconstitute the bioprosthetic tissue by increasing its moisture content at least partially outside of the patient's body and/or fully *in situ* (in the patient's body) via the patient's biofluids. The "dry" tissue valve may be reconstituted at least partially in that the tissue valve may be briefly exposed to a reconstituting solution, and then immediately implanted prior to complete reconstitution. Alternatively, the tissue valve may be exposed to the reconstituting solution for a period long enough to completely reconstitute the moisture content of the tissue valve to the point that it is substantially similar to the moisture content of the tissue valve as it would exist *in situ.* The reconstitution solution may be any fluid that transfers moisture into the "dry" tissue, for example, a saline solution with a 0.9% concentration of NaCl.

FIG. 5 illustrates several embodiments of a method 400 of reconstituting a "dry" tissue valve that has been pre-loaded and delivered to the surgical team in the pre-loaded configuration. Such a method generally includes reconstituting by immediate implantation of the "dry" tissue valve, reconstituting while the "dry" tissue valve remains fully sheathed by the delivery device, and reconstituting while partially unsheathed and exposed to a reconstituting solution outside of the patient's body or a biofluid within the patient's body.

According to a first embodiment 410 shown in FIG. 5, the surgical team in receipt of a "dry" non-resheathable tissue valve in the pre-loaded configuration may guide a non-resheathable delivery device to the diseased valve through the patient's cardiovascular system or trans-apically wherein a "dry" non-resheathable tissue valve is immediately implanted. In other words, the "dry" non-resheathable tissue valve is implanted as delivered to the surgical team. Thus, the surgical team may implant 412 the "dry" non-resheatable tissue valve into the native annulus wherein the "dry" valve assembly begins to take over the valvular functions. Immediately after implantation, the "dry" tissue begins to absorb moisture from the patient's biofluid, thereby beginning the reconstitution process of the bioprosthetic tissue, which is ultimately fully reconstituted *in situ* 414. The "dry" tissue as implanted may be effective to sufficiently perform the vavlular functions. However, the valve's performance generally increases to its peak performance level at full reconstitution.

According to another embodiment 420, the "dry" tissue valve may be at least partially rehydrated or reconstituted while it is fully sheathed within the delivery device. The delivery device may have a fluid channel extending from a fluid source into at least the location in which the tissue valve is located. Thus, a fluid may be brought 422 into contact with the tissue valve while the tissue valve remains fully sheathed. This fluid contact may occur either outside the patient or inside the patient during the implantation process before final implantation. The at least partially reconstituted tissue valve may thereafter be implanted 424. In the event the tissue valve has not been fully reconstituted prior to implantation, the tissue valve may fully reconstitute 426 via the patient's biofluids *in situ.*

In yet another embodiment 430, the "dry" tissue valve may be either at least partially reconstituted inside the patient's body or outside the patient's body. As previously described, the delivery device may be capable of partially unsheathing the "dry" tissue valve so that the bioprosthetic tissue may be exposed directly to a reconstituting fluid.

In one aspect of embodiment 430, the surgical team in receipt of the pre-loaded tissue valve may partially unsheathe 431 the "dry" tissue valve and either soak the tissue valve in a reconstituting solution until the bioprosthetic tissue reaches complete reconstitution 432, or the operator may briefly expose the "dry" tissue valve to the reconstituting solution in order to provide partial reconstitution of the tissue valve prior to implantation, wherein the bioprosthetic tissue 432. Once reconstitution of the "dry" tissue valve has at least commenced, it may be resheathed 433 in preparation for implantation.

In another aspect of embodiment, the operator may introduce the pre-loaded delivery device to the patient's cardiovascular system. At the implantation location, or before then, the "dry" tissue valve may be partially unsheathed 431 exposing the "dry" tissue to the patient's biofluids to begin the reconstitution process. The at least partially reconstituted tissue valve may thereafter be fully resheathed 433, perhaps to relocate the tissue valve, or it could be directly implanted.

Another method 500 of reconstitution is illustrated by FIG. 6. It does not form part of the invention. This method generally includes the "dry" tissue valve received by the surgical team in the free-standing configuration. The "dry" tissue valve in this configuration may generally be reconstituted by either loading the delivery device as received and reconstituting *in situ* or at least partially reconstituting prior to loading the delivery device.

In a first embodiment, the surgical team in receipt of the "dry" tissue valve in the free-standing configuration may crimp and load 512 the delivery device, which may either be of a reduced profile due to lyophilization or may be a standard size delivery device. The loaded delivery device may thereafter be introduced to the patient's cardiovascular system and navigated to the implantation located wherein the "dry" tissue valve is fully deployed and implanted 514. As has been already described, the "dry" tissue valve may be fully reconstituted 516 once implanted into the annulus if complete reconstitution has not yet be achieved. Additionally, it is noted that the tissue valve may be partially unsheathed *in situ* in order to verify operability or positional alignment, and or to assist in reconstitution thereby at least partially reconstituting the bioprosthetic tissue prior to implantation.

In another embodiment 520, the "dry" tissue valve in the free-standing configuration may be at least partially reconstituted 522 outside the patient's body by either soaking or briefly exposing the tissue valve to a reconstituting solution. This may be done to increase the bioprosthetic tissue's suppleness or the verify operability. Thereafter, the at least partially reconstituted tissue valve may be crimped and loaded 524 into the delivery device and introduced into the patient's cardiovascular system where it is implanted 524 and reconstituted 528. Similar to embodiment, the tissue valve may be at least partially unsheathed during the implantation process, which may serve to further reconstitute the bioprosthetic tissue.

Although the invention herein has been described with reference to particular embodiments, it is to be understood that these embodiments are merely illustrative of the principles and applications of the present invention. It is therefore to be understood that numerous modifications may be made within the scope of the appended claims.

### INDUSTRIAL APPLICABILITY

The present invention enjoys wide industrial applicability including, but not limited to, providing tissue valves that include lyophilized tissue and methods of making the same.

## Claims

1. A method of making a tissue valve (10), comprising:
attaching a valve assembly within a support (20) configured to fit within a blood vessel; and
lyophilizing the valve assembly after the valve assembly has been attached to the support.

2. The method of claim 1, wherein the support is a stent that includes an annulus section (23), an aortic section (24), a plurality of commissure features (25), a proximal end (21), and a distal end (22), and the attaching step includes attaching the valve assembly to the annulus section.

3. The method of claim 1, wherein the valve assembly (30) includes a cuff (31) and at least one leaflet (32), the method further comprising connecting the at least one leaflet to the cuff prior to the attaching step.

4. The method of claim 1, wherein the valve assembly includes a cuff and at least one leaflet, the method further comprising attaching the cuff to the support and then sewing the at least one leaflet to the cuff.

5. The method of claim 1, wherein the lyophilizing step is performed until the valve assembly has a moisture content of 10wt% or less.

6. The method of claim 1, wherein the lyophilizing step is performed until the valve assembly has a moisture content of 5wt% or less.

7. The method of claim 1, wherein the lyophilizing step is performed until the valve assembly has a moisture content of 1wt% or less.

8. The method of claim 1, further comprising soaking the valve assembly in a saline solution prior to the lyophilizing step.

9. The method of claim 1, further comprising treating the valve assembly with a lyoprotectant or a cryoprotectant before the lyophilization step.

10. The method of claim 1, wherein the lyophilizing step includes:
freezing the valve assembly at about -20 degrees C for at least 12 hours; and
vacuum drying the valve assembly at room temperature for 15-20 hours.

11. The method of claim 1, wherein the lyophilizing step includes:
freezing the valve assembly at about -70 degrees C for at least 2 hours;
annealing the valve assembly at about -20 degrees C for at least 1 hour;
refreezing the valve assembly at about -70 degrees C for at least 2 hours; and
drying the valve assembly at a pressure of about 750 mTorr.

12. The method of claim 1, wherein the lyophilizing step includes:
freezing the valve assembly to about -40 degrees C;
annealing the valve assembly at about -20 degrees C for at least 1 hour;
refreezing the valve assembly to about -40 degrees C for at least 2 hours;
primary drying the valve assembly at about -5 degrees C at a pressure of about 160 mTorr; and
secondary drying the valve assembly at 20-25 degrees C at a pressure of about 160 mTorr for at least 2 hours.

13. The method as in any one of claims 10, 11, and 12, further comprising treating the valve assembly with a lyoprotectant including a mixture of sucrose and hydroxyl ethylene starch (HES).

14. The method of claim 13, wherein the concentration of sucrose is at least 1.5% and the concentration of HES is at least 1.5%.

15. The method as in any one of claims 10, 11, and 12, further comprising treating the valve assembly with a lyoprotectant including sucrose at a concentration of at least 2%.

## Patentansprüche

1. Verfahren zur Erzeugung einer Gewebeklappe (10), das Folgendes umfasst:
Anbringen einer Klappenanordnung innerhalb einer Halterung (20), die zum Einpassen innerhalb eines Blutgefäßes konfiguriert ist; und
Lyophilisieren der Klappenanordnung nachdem die Klappenanordnung an die Halterung angebracht wurde.

2. Verfahren nach Anspruch 1, wobei die Halterung ein Stent ist, der einen Anulusabschnitt (23), einen Aortenabschnitt (24), eine Vielzahl von Kommissurenmerkmalen (25), ein proximales Ende (21) und ein distales Ende (22) einschließt,
und der Anbringungsschritt das Anbringen der Klappenanordnung an den Anulusabschnitt einschließt.

3. Verfahren nach Anspruch 1, wobei die Klappenanordnung (30) einen Cuff (31) und mindestens ein Segel (32) einschließt, das Verfahren weiter das Verbinden des mindestens einen Segels mit dem Cuff vor dem Anbringungsschritt umfasst.

4. Verfahren nach Anspruch 1, wobei die Klappenanordnung einen Cuff und mindestens ein Segel einschließt, das Verfahren weiter das Anbringen des Cuffs an die Halterung und dann das Nähen des mindestens einen Segels an den Cuff umfasst.

5. Verfahren nach Anspruch 1, wobei der Lyophilisierungsschritt durchgeführt wird, bis die Klappenanordnung einen Feuchtigkeitsgehalt von 10 Gew.-% oder weniger aufweist.

6. Verfahren nach Anspruch 1, wobei der Lyophilisierungsschritt durchgeführt wird, bis die Klappenanordnung einen Feuchtigkeitsgehalt von 5 Gew.-% oder weniger aufweist.

7. Verfahren nach Anspruch 1, wobei der Lyophilisierungsschritt durchgeführt wird, bis die Klappenanordnung einen Feuchtigkeitsgehalt von 1 Gew.-% oder weniger aufweist.

8. Verfahren nach Anspruch 1, das weiter das Einweichen der Klappenanordnung in einer Kochsalzlösung vor dem Lyophilisierungsschritt umfasst.

9. Verfahren nach Anspruch 1, das weiter das Behandeln der Klappenanordnung mit einem Lyoprotektivum oder einem Kryoprotektivum vor dem Lyophilisierungsschritt umfasst.

10. Verfahren nach Anspruch 1, wobei der Lyophilisierungsschritt Folgendes einschließt:
Gefrieren der Klappenanordnung bei etwa -20 Grad C für mindestens 12 Stunden und
Vakuumtrocknen der Klappenanordnung bei Raumtemperatur für 15-20 Stunden.

11. Verfahren nach Anspruch 1, wobei der Lyophilisierungsschritt Folgendes einschließt:
Gefrieren der Klappenanordnung bei etwa -70 Grad C für mindestens 2 Stunden,
Tempern der Klappenanordnung bei etwa -20 Grad C für mindestens 1 Stunde,
erneutes Gefrieren der Klappenanordnung bei etwa -70 Grad C für mindestens 2 Stunden und
Trocknen der Klappenanordnung bei einem Druck von etwa 750 mTorr.

12. Verfahren nach Anspruch 1, wobei der Lyophilisierungsschritt Folgendes einschließt:
Gefrieren der Klappenanordnung bei etwa -40 Grad C,
Tempern der Klappenanordnung bei etwa -20 Grad C für mindestens 1 Stunde,
erneutes Gefrieren der Klappenanordnung auf etwa -40 Grad C für mindestens 2 Stunden und
primäres Trocknen der Klappenanordnung bei etwa -5 Grad C bei einem Druck von etwa 160 mTorr und
sekundäres Trocknen der Klappenanordnung bei 20-25 Grad C bei einem Druck von etwa 160 mTorr für mindestens 2 Stunden.

13. Verfahren nach einem der Ansprüche 10, 11 und 12, das weiter das Behandeln der Klappenanordnung mit einem Lyoprotektivum umfasst, das eine Mischung aus Saccharose und Hydroxylethylen-Stärke (HES) einschließt.

14. Verfahren nach Anspruch 13, wobei die Konzentration von Saccharose mindestens 1,5 % beträgt und die Konzentration von HES mindestens 1,5 % beträgt.

15. Verfahren nach einem der Ansprüche 10, 11 und 12, das weiter das Behandeln der Klappenanordnung mit einem Lyoprotektivum umfasst, das Saccharose in einer Konzentration von mindestens 2 % einschließt.

## Revendications

1. Procédé de fabrication d'une valve en tissu (10), comprenant :
attacher un ensemble de valve à l'intérieur d'un support (20) configuré pour s'adapter dans un vaisseau sanguin ; et
lyophiliser l'ensemble de valve après que l'ensemble de valve a été attaché au support.

2. Procédé selon la revendication 1, dans lequel le support est un stent qui comprend une section d'anneau (23), une section aortique (24), une pluralité d'éléments de commisure (25), une extrémité proximale (21) et une extrémité distale (22),
et l'étape d'attache comprend attacher l'ensemble de valve à la section d'anneau.

3. Procédé selon la revendication 1, dans lequel l'ensemble de valve (30) comprend une manchette (31) et au moins un feuillet (32),
le procédé comprenant en outre raccorder le au moins un feuillet à la manchette préalablement à l'étape d'attache.

4. Procédé selon la revendication 1, dans lequel l'ensemble de valve comprend une manchette et au moins un feuillet, le procédé comprenant en outre attacher la manchette au support et puis coudre le au moins un feuillet à la manchette.

5. Procédé selon la revendication 1, dans lequel l'étape de lyophilisation est effectuée jusqu'à ce que l'ensemble de valve ait une teneur en humidité de 10 % en poids ou moins.

6. Procédé selon la revendication 1, dans lequel l'étape de lyophilisation est effectuée jusqu'à ce que l'ensemble de valve ait une teneur en humidité de 5 % en poids ou moins.

7. Procédé selon la revendication 1, dans lequel l'étape de lyophilisation est effectuée jusqu'à ce que l'ensemble de valve ait une teneur en humidité de 1 % en poids ou moins.

8. Procédé selon la revendication 1, comprenant en outre tremper l'ensemble de valve dans une solution saline préalablement à l'étape de lyophilisation.

9. Procédé selon la revendication 1, comprenant en outre traiter l'ensemble de valve avec un agent lyoprotecteur ou un agent cryoprotecteur préalablement à l'étape de lyophilisation.

10. Procédé selon la revendication 1, dans lequel l'étape de lyophilisation comprend :
congeler l'ensemble de valve à environ - 20 degrés C pendant 12 heures au moins ; et
sécher sous vide l'ensemble de valve à température ambiante pendant 15 - 20 heures.

11. Procédé selon la revendication 1, dans lequel l'étape de lyophilisation comprend :
congeler l'ensemble de valve à environ - 70 degrés C pendant 2 heures au moins ;
recuire l'ensemble de valve à environ - 20 degrés C pendant 1 heure au moins ;
recongeler l'ensemble de valve à environ - 70 degrés C pendant 2 heures au moins ; et
sécher l'ensemble de valve à une pression d'environ 750 mTorr.

12. Procédé selon la revendication 1, dans lequel l'étape de lyophilisation comprend :
congeler l'ensemble de valve à environ - 40 degrés C ;
recuire l'ensemble de valve à environ - 20 degrés C pendant 1 heure au moins ;
recongeler l'ensemble de valve à environ - 40 degrés C pendant 2 heures au moins ;
effectuer un séchage primaire de l'ensemble de valve à environ - 5 degrés C à une pression d'environ 160 mTorr ; et
effectuer un séchage secondaire de l'ensemble de valve à 20 - 25 degrés C à une pression d'environ 160 mTorr pendant 2 heures au moins.

13. Procédé selon l'une quelconque des revendications 10, 11 et 12, comprenant en outre traiter l'ensemble de valve avec un agent lyoprotecteur comprenant un mélange de saccharose et d'amidon hydroxéthylé (HES).

14. Procédé selon la revendication 13, dans lequel la concentration de saccharose est d'au moins 1, 5% et la concentration de HES est d'au moins 1,5 %.

15. Procédé selon l'une quelconque des revendications 10, 11 et 12, comprenant en outre traiter l'ensemble de valve avec un agent lyoprotecteur comprenant du saccharose à une concentration d'au moins 2 %.
